# FASCICULE DE BREVET EUROPEEN

(11) **EP 0 701 834 B1**
(45) Date de publication et mention de la délivrance du brevet: **27.10.1999**
(21) Numéro de dépôt: 95402014.5
(22) Date de dépôt: 06.09.1995
(51) Int. Cl.: A61M 16/00, A61M 39/22

(54) **Dispositif d'assistance respiratoire**
Vorrichtung zur Unterstützung der Atmung
Respiratory assistance device

(30) Priorité: 16.09.1994 FR 9411062
(43) Date de publication de la demande: 20.03.1996
(73) Titulaire: Boussignac, Georges, 92160 Antony (FR); Labrune, Jean-Claude, F-92310 Sèvres (FR)
(72) Inventeur: Boussignac, Georges, 92160 Antony (FR); Labrune, Jean-Claude, F-92310 Sèvres (FR)
(74) Mandataire: Bonnetat, Christian

(56) Documents cités:
- EP-A- 0 042 321
- EP-A- 0 390 684
- WO-A-93/21982
- US-A- 3 717 174
- US-A- 5 050 593
- US-A- 5 161 773
- US-A- 5 186 431

## Description

La présente invention a pour objet un dispositif pour assistance respiratoire, utilisable sur des patients dont la respiration spontanée est absente ou insuffisante, qu'ils soient placés ou non sous respiration artificielle.

On connaît divers dispositifs, tels que des masques, des sondes ou des canules orales, nasales, endotrachéales, trachéotomiques, destinés à faire la jonction entre un appareil de respiration artificielle et/ou d'anesthésie et le système respiratoire d'un patient. Ces dispositifs, essentiellement en forme de tubes, peuvent, selon le cas, comporter des moyens d'immobilisation tels que des pattes ou des collerettes au voisinage de l'extrémité proximale, pour le maintien sur la bouche ou le nez du patient, ou encore des ballonnets gonflables au voisinage de l'extrémité distale, pour le maintien par friction dans la trachée.

Les dispositifs connus présentent des inconvénients importants. Ainsi, par exemple, lorsqu'un tube de type connu est déconnecté du respirateur artificiel et que le patient a besoin d'air enrichi en oxygène, il est nécessaire d'introduire dans ledit tube une sonde reliée à une source d'oxygène. Par ailleurs, dans les cas de respiration spontanée insuffisante, le patient doit nécessairement rester relié au respirateur jusqu'au rétablissement complet de sa respiration spontanée.

Aussi, pour remédier à ces inconvénients, on a déjà proposé des dispositifs d'assistance respiratoire qui, outre le canal principal formé par le tube, comportent au moins un canal auxiliaire, par exemple ménagé dans la paroi dudit tube, permettant l'injection d'un jet de gaz respirable (oxygène, air ou mélange air-oxygène) destiné à la ventilation du patient, ce canal auxiliaire débouchant dans le canal principal au voisinage de l'extrémité distale de ce dernier.

Toutefois, ces dispositifs d'assistance respiratoire à canal auxiliaire d'injection de gaz respiratoire présentent l'inconvénient que ledit jet de gaz respirable doit être à une pression élevée, ce qui, d'une part, risque de traumatiser la muqueuse du patient qu'elle frappe et, d'autre part, rend difficile, sinon impossible, l'humidification dudit gaz respirable. On sait en effet qu'un jet gazeux est d'autant plus difficile à humidifier que sa pression est plus élevée. Or, il est important que le jet de gaz respirable soit humidifié afin d'éviter le dessèchement de la muqueuse du patient.

La présente invention a pour objet principal de remédier à cet inconvénient.

A cette fin, selon l'invention, le dispositif d'assistance respiratoire comportant un tube qui forme un canal principal et qui est destiné à être relié par son extrémité distale à une voie respiratoire d'un patient pour que ledit canal principal relie, à l'extérieur, le système respiratoire dudit patient, ledit dispositif comportant de plus au moins un canal auxiliaire permettant l'injection d'un jet gazeux respirable dans ledit système respiratoire et débouchant dans ledit canal principal au voisinage de l'extrémité distale de ce dernier, est remarquable en ce qu'il comporte une vanne commandée susceptible d'obturer au moins partiellement l'extrémité proximale dudit canal principal, au moins pendant l'insufflation dudit jet gazeux.

Ainsi, du fait que ladite extrémité proximale du canal principal est au moins partiellement obturée pendant l'insufflation du jet gazeux respirable, la pression de celui-ci peut être abaissée (puisque la fuite de gaz vers l'extérieur est empêchée ou, pour le moins, réduite) pour un effet de ventilation semblable. L'abaissement de la pression du gaz de ventilation permet alors l'humidification aisée dudit gaz, par exemple par barbotage ou bien par pulvérisation.

Bien entendu, ledit jet gazeux respirable peut, de façon connue, être continuel ou impulsionnel. Si ledit jet gazeux est continuel, ladite vanne commandée doit, d'une part, fermer partiellement ou totalement l'extrémité proximale dudit canal principal pendant l'inspiration du patient, afin d'obtenir l'abaissement de pression précité et, d'autre part, ouvrir partiellement ou totalement ladite extrémité proximale pendant l'expiration dudit patient. L'ouverture partielle de cette extrémité proximale pendant l'expiration du patient peut être utilisée comme "frein expiratoire" permettant d'obliger ledit patient à des exercices bénéfiques de respiration. Si, en revanche, ledit jet gazeux est impulsionnel, ladite vanne commandée doit, d'une part, fermer partiellement ou totalement l'extrémité proximale dudit canal principal pendant les impulsions du jet gazeux, à la même fin d'abaissement de pression que précédemment, et, d'autre part, ouvrir partiellement ou totalement ladite extrémité proximale entre lesdites impulsions du jet gazeux. Entre ces impulsions, il est bien entendu possible de se servir de l'ouverture partielle de ladite vanne commandée pour obtenir un "frein expiratoire".

On remarquera que l'on peut même utiliser le dispositif conforme à l'invention sans insufflation de gaz respirable, le patient inspirant et expirant à travers ledit canal et ladite vanne commandée, qui commande alors la section de passage dudit canal.

Ladite vanne commandée peut former un ensemble monolithique avec ledit tube ou bien elle peut être rapportée à celui-ci. Dans ce dernier cas, qui permet de transformer des tubes respiratoires normaux en tubes respiratoires conformes à la présente invention, il est avantageux que ladite vanne commandée soit solidaire d'un embout susceptible d'être emboîté sur l'extrémité proximale dudit tube.

La vanne commandée peut être de toute réalisation connue, avec commande électrique, pneumatique, etc ... Cependant, il est avantageux qu'elle permette, en position ouverte, de conserver au canal principal un diamètre intérieur suffisant pour permettre le passage à travers ledit canal des instruments et des sondes médicales que l'opérateur veut utiliser, sans interrompre la ventilation.

Aussi, dans un mode de réalisation préféré, ladite vanne commandée comporte une enceinte étanche à section toroïdale, disposée au voisinage de l'extrémité proximale dudit canal principal et comportant au moins une paroi interne souple et élastique qui, en se dilatant ou en se rétractant en réponse à l'introduction ou à l'évacuation d'un gaz de gonflage dans ladite enceinte étanche, commande la section de passage dudit canal principal. On remarquera qu'une telle vanne, même lorsqu'elle est complètement fermée, permet le passage d'instruments ou de sondes dans ledit canal par l'écartement forcé de ladite paroi interne souple.

Afin d'éviter qu'un objet appliqué par inadvertance contre l'orifice proximal dudit tube obture ledit canal principal et empêche l'expiration du patient, le bord libre de l'extrémité proximale dudit tube comporte, dans un mode de réalisation préféré, au moins une échancrure.

Egalement à des fins de sécurité, le dispositif conforme à la présente invention comporte de plus, dans un mode de réalisation préféré, des moyens recevant la pression du côté de l'extrémité distale dudit tube et susceptibles d'imposer l'ouverture à ladite vanne commandée pour dégager ledit canal principal. Pour augmenter encore la sécurité, on peut prévoir une vanne d'échappement tarée disposée à l'extrémité proximale dudit tube, du côté opposé au bord libre de l'extrémité proximale par rapport à ladite vanne commandée.

Pour augmenter encore plus la sécurité, on peut faire en sorte que l'introduction et l'évacuation du gaz de gonflage de ladite enceinte étanche résulte du déplacement bidirectionnel d'un volume de gaz approprié, contenu dans une capacité tampon à volume variable commandable.

Par ailleurs, pour éviter que le jet de gaz respirable humidifié vienne frapper directement la muqueuse, risquant par son énergie cinétique de traumatiser la muqueuse, il est avantageux que, comme cela a été décrit dans le brevet européen EP-A-0 390 684, au moins l'extrémité distale dudit canal auxiliaire débouchant dans le canal principal soit parallèle à celui-ci et que, en regard de l'orifice distal dudit canal auxiliaire, soient prévus des moyens de déflexion dudit jet de gaz respirable de ventilation vers l'intérieur dudit canal principal.

Ainsi, le jet de gaz respirable humidifié sous faible pression traversant ledit canal auxiliaire est défléchi vers l'axe du canal principal, lorsqu'il pénètre dans celui-ci. En aval desdits moyens de déflexion, c'est-à-dire à l'intérieur du canal principal, la pression dudit jet de gaz respirable chute et le jet sort à encore plus faible pression à travers l'orifice distal du tube. L'expérience a montré qu'en aval de la sortie distale du tube, la pression est faible et maintenue constante dans tout l'espace respiratoire. Cette pression est dépendante du débit de gaz respirable dans le canal auxiliaire. Par suite, avec le dispositif d'assistance respiratoire conforme à l'invention, on peut par exemple apporter de l'oxygène humide ou un mélange air-oxygène humide directement dans les poumons, à hauteur de la carène, et supprimer ainsi l'espace mort qui existe dans les sondes actuelles et qui est d'environ un tiers du volume respiratoire total pour un adulte et d'environ la moitié pour les nouveaux-nés prématurés.

La suppression de cet espace mort correspond à une augmentation de performance du cycle respiratoire de plus de 25% dans tous les cas de malades et de près de 50% dans certains cas.

Lorsque le dispositif selon l'invention comporte une pluralité de canaux auxiliaires, il est avantageux qu'au moins certains d'entre eux soient alimentés en commun en gaz respirable. Une telle alimentation en commun desdits canaux peut se faire par l'intermédiaire d'une bague de distribution, coaxiale audit tube. Par ailleurs, lesdits canaux auxiliaires non alimentés en commun peuvent servir à l'introduction de produits gazeux additionnels, tels que des produits médicamenteux.

Ainsi, on voit que le dispositif conforme à l'invention, au moins dans son mode de réalisation préféré, permet :
- l'humidification du gaz respirable insufflé,
- l'intubation de longue durée de l'assistance respiratoire sans assèchement,
- l'injection de médicaments ou d'anesthésiques pendant l'assistance respiratoire,
- la mesure dynamique des pressions, car il suffit de prévoir des canaux auxiliaires auxquels sont associées des sondes appropriées,
- l'établissement d'un microdébit de gaz respirable dans les canaux auxiliaires pour éviter l'obstruction desdits canaux par des mucosités ;
- l'augmentation du volume échangé, car la pression est auto-limitée et il n'y a aucun risque d'écrasement des capillaires pulmonaires,
- la diminution pour une même quantité d'oxygène échangée de l'importance d'oxygène dans le mélange, ce qui diminue d'autant les effets secondaires de l'assistance,
- la possibilité d'utiliser des respirateurs moins coûteux que les respirateurs actuels.

Les figures du dessin annexé feront bien comprendre comment l'invention peut être réalisée. Sur ces figures, des références identiques désignent des éléments semblables.
La figure 1 est une vue schématique et partielle, en coupe axiale agrandie, d'un mode de réalisation du dispositif de l'invention.
Les figures 2 et 3 sont des coupes transversales, respectivement selon les lignes II-II et III-III de la figure 1.
La figure 4 illustre, schématiquement en coupe axiale agrandie, un mode de réalisation préféré pour la vanne disposée à l'extrémité proximale du dispositif, conformément à l'invention.
La figure 5 montre, en perspective schématique et partielle, l'extrémité proximale du dispositif conforme à l'invention.
La figure 6 illustre schématiquement et partiellement une variante de réalisation du dispositif respiratoire conforme à la présente invention.
Les figures 7A et 7B illustrent schématiquement un dispositif de commande de la fermeture et de l'ouverture des vannes des figures 4 et 6.

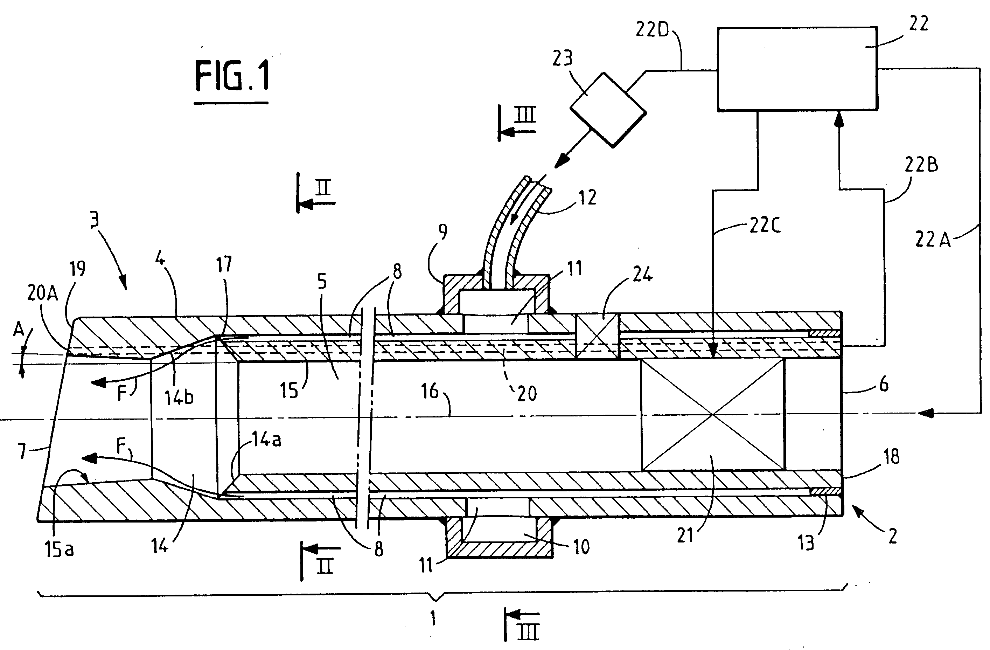

Sur la figure 1, on a représenté, schématiquement et à grande échelle, les seules extrémités proximale 2 et distale 3 d'un mode de réalisation monolithique 1 du dispositif selon l'invention. Ce mode de réalisation monolithique peut constituer, par exemple, une sonde endotrachéale oro-nasale avec ou sans ballonnet, une sonde endotrachéale pédiatrique, une sonde de monitorage des gaz, une sonde endobronchique, une sonde nasopharyngée, une sonde d'intubation anatomique pour enfant, une sonde de Cole néonatale, une sonde canule de Gedel, une sonde nasale d'oxygénothérapie, un masque nasal ou bucconasal ou un ballon nasal pour traitement d'apnée du sommeil.

Le dispositif 1 comporte un tube 4, souple ou préformé (pour s'adapter à la morphologie du patient) délimitant un canal principal 5 débouchant, par l'orifice 6, à l'extrémité proximale 2 et, par l'orifice 7, à l'extrémité distale 3.

Ainsi, le canal principal 5 est capable d'assurer le passage entre les orifices 6 et 7, dont l'un (l'orifice 7) est destiné à se trouver à l'intérieur des voies respiratoires d'un patient, et l'autre (l'orifice 6) est destiné à se trouver à l'extérieur dudit patient. Cet orifice 6 peut déboucher à l'air libre et, dans ce cas, le patient peut inspirer de l'air frais et expirer l'air vicié à travers le canal principal 5. On peut également, comme cela est expliqué ci-après, relier l'orifice 6 à une source de gaz respirable sous pression et prévoir un système de valves unidirectionnelles, pour que le patient inspire le gaz respirable de ladite source à travers ledit canal principal 5 et expire le gaz vicié à l'air libre, également à travers ce canal principal.

Le diamètre du canal principal 5 est de l'ordre de quelques millimètres. Des essais satisfaisants ont été effectués avec des diamètres de 3 mm, 7 mm, 8 mm et 12 mm.

Par ailleurs, dans l'épaisseur de la paroi du tube 4, sont ménagés des canaux auxiliaires 8, s'étendant sur la presque totalité de la longueur du canal principal 5. Ces canaux auxiliaires 8 sont destinés à être reliés à une source de gaz respirable sous pression. Par exemple, cette pression est au plus égale à 0,5 bar et elle est réglable.

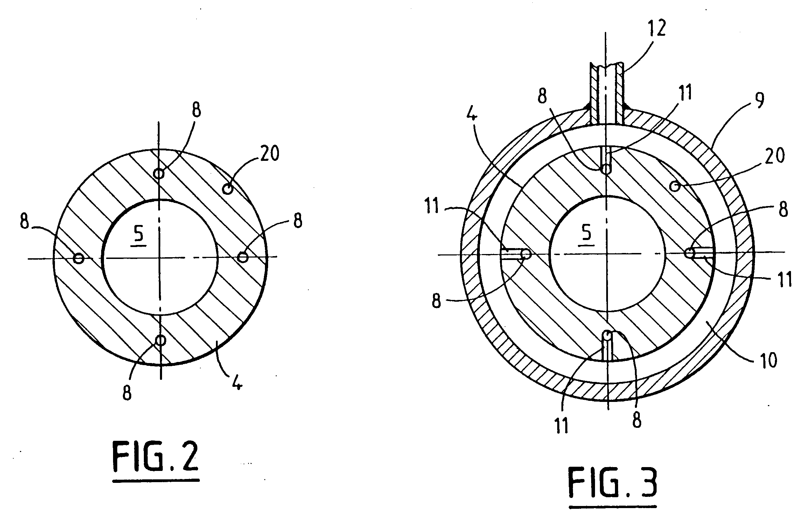

Comme cela est représenté sur les figures 1 et 3, la liaison à la source de gaz respirable sous pression peut être réalisée au moyen d'une bague 9, entourant de façon étanche le tube 4, du côté de l'extrémité proximale 2, et délimitant une chambre annulaire étanche 10 autour dudit tube. Les canaux auxiliaires 8 sont mis en communication avec la chambre 10, grâce à des arrachements locaux 11 de la paroi du tube 4, et ladite chambre 10 est reliée à ladite source de gaz respirable par un conduit 12. Bien entendu, les extrémités proximales des canaux 8 sont obturées, par exemple par des bouchons 13, introduits à partir de la face d'extrémité proximale 18 du tube 4.

Les canaux auxiliaires 8 ont un diamètre plus petit que celui du canal principal 5. Le diamètre des canaux auxiliaires 8 est de préférence inférieur à 1 mm et, de façon avantageuse, il est de l'ordre de 400 à 800 microns. Du côté distal, les canaux auxiliaires 8 débouchent dans un évidement 14 de la paroi interne 15 du tube 4. L'évidement 14 est annulaire et centré sur l'axe 16 de l'extrémité distale 3. Il comporte une face 14a, sensiblement transversale ou légèrement inclinée de façon à constituer un évasement du canal principal 5, dans laquelle débouchent lesdits canaux auxiliaires 8 par leurs orifices 17, ainsi qu'une face 14b suivant la face 14a et convergeant en direction de l'axe 16.

De préférence, entre la face inclinée convergente 14b et l'orifice distal 7, la paroi interne 15 présente une partie 15a légèrement évasée vers l'extérieur, comme cela est illustré par l'angle A sur la figure 1.

Ainsi, lorsque les canaux auxiliaires 8 sont alimentés en gaz respirable sous pression à travers les éléments 9 à 12, les jets gazeux correspondants heurtent la face inclinée 14b, qui les défléchit en direction de l'axe 16 (flèches F sur la figure 1), engendrant au voisinage de celui-ci une zone de dépression favorisant la circulation gazeuse à l'intérieur du canal principal 5, de l'orifice proximal vers l'orifice distal. On favorise ainsi l'inspiration du patient.

De préférence, la distance entre chacun des orifices 17 et l'orifice 7 est de l'ordre de 1 à 2 cm.

Au moins un canal supplémentaire 20 est prévu dans l'épaisseur du tube 4 afin de déboucher en 20A au voisinage de l'extrémité distale 19 du tube 4 et servir de prise de pression.

Le dispositif 1 comporte de plus une vanne commandée 21 susceptible d'obturer au moins partiellement l'extrémité proximale 2 du canal principal 5 pendant l'insufflation dudit jet gazeux, ainsi qu'un dispositif d'alimentation et de commande 22. Ce dernier dispositif 22 est relié :
- à l'extrémité proximale 2 du tube 4, par une liaison 22A,
- au canal supplémentaire 20, par une liaison 22B,
- à la vanne commandée 21, par une liaison 22C, et
- au conduit 12, par une liaison 22D sur laquelle est intercalé un humidificateur 23.

Les modes de fonctionnement du dispositif selon l'invention sont les suivants :
- dans le mode de respiration artificielle, le dispositif 22 coupe la liaison 22D, commande la vanne 21 à l'ouverture au moins partielle par la liaison 22C et adresse du gaz respirable dans le tube 4 par l'intermédiaire de la liaison 22A ;
- dans le mode d'assistance respiratoire, le dispositif 22 coupe la liaison 22A pour mettre l'orifice 6 en communication avec l'atmosphère, commande la vanne 21 alternativement à l'ouverture et à la fermeture par la liaison 22C et adresse un jet, continuel ou impulsionnel, de gaz respirable aux canaux auxiliaires 8 par la liaison 22D, à travers l'humidificateur 23. Dans ce mode d'assistance respiratoire, la vanne 21 est fermée, partiellement ou totalement, pendant l'insufflation de gaz respirable dans le patient à travers les canaux 8 et ouverte au moins partiellement pendant l'expiration dudit patient à travers le tube 4, vers l'extérieur. Du fait que l'extrémité proximale 2 du tube 4 est obturée pendant l'insufflation de gaz respirable, la pression de celui-ci peut être faible, ce qui permet de l'humidifier facilement dans l'humidificateur 23.

Dans les deux modes de fonctionnement, le dispositif d'alimentation et de commande 22 est informé de la pression régnant dans les poumons du patient par l'intermédiaire de la liaison 22B. Dans le cas d'une surpression anormale, le dispositif 22 peut ainsi déclencher une alarme et arrêter l'insufflation de gaz respirable à travers la liaison 22A ou la liaison 22D.

Pour augmenter encore la sécurité, une vanne d'échappement tarée 24 peut être prévue dans l'extrémité proximale 2 du tube 4, du côté opposé à l'orifice 6 par rapport à la vanne commandée 21. Ainsi, en cas de surpression accidentelle, une fuite de gaz se produit à l'extérieur du patient, à travers la paroi du tube 4, pour éliminer instantanément cette surpression.

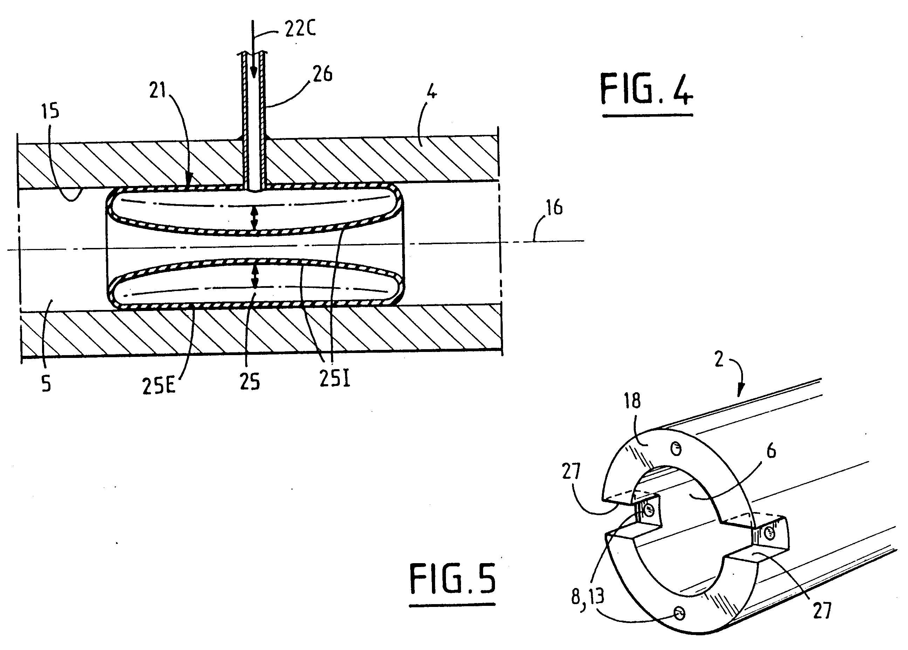

Sur la figure 4, on a illustré schématiquement un mode de réalisation préféré pour ladite vanne commandée 21. Dans ce mode de réalisation, la vanne commandée 21 comporte une vessie gonflable élastique 25 à section toroïdale, dont la paroi externe 25E prend appui contre la paroi interne 15 du canal principal 5 et qui est reliée à la liaison 22C par un conduit 26. Ainsi, du gaz de gonflage adressé à ladite vessie 25 par le dispositif 22 permet à la paroi interne 25I de celle-ci de se rapprocher, de façon à restreindre ou obturer le canal principal, tandis que ladite vessie se rétracte spontanément sous l'action de sa propre élasticité, lorsque ledit gaz de gonflage est évacué de la vessie, en dégageant ledit canal principal (sur la figure 4, la vessie 25 est représentée en position moyenne entre l'ouverture et la fermeture).

Lorsque cette vanne 21 est en position ouverte, la paroi interne 25I de la vessie 25 est écartée et ménage suffisamment de place pour passer des appareils médicaux et chirurgicaux à travers le tube 4. On remarquera que, même en position fermée, de tels appareils médicaux et chirurgicaux peuvent être passés de force à travers la vanne 21, grâce à l'élasticité de la paroi interne 25I.

Comme le montre la figure 5, la face annulaire proximale 18 délimitant l'orifice proximal 6 du tube 4 est pourvue d'échancrures 27 permettant la communication du canal principal 5 avec l'extérieur, même si l'orifice 6 est bouché par un objet qui s'y appuie accidentellement.

De ce qui précède, on voit donc qu'on obtient une assistance respiratoire humidifiée non agressive pour le patient, avec disparition quasiment totale de l'espace mort inhérent aux sondes connues.

Le tube 4 peut comporter, à l'extrémité distale 3, un ballonnet gonflable (non représenté) muni des dispositifs nécessaires de sécurité ou tout autre ballonnet permettant de se comporter comme une soupape de sécurité en cas de surpression dans les poumons. Cet éventuel ballonnet peut être gonflé à partir d'un canal supplémentaire (non représenté) associé au tube 4.

De plus, un au moins des canaux auxiliaires 8 peut être spécialisé pour apporter un fluide médical.

Comme le montrent les figures 2 et 3, les canaux auxiliaires 8 sont disposés régulièrement autour de l'axe du tube 4. Leur nombre est variable suivant les utilisations (adulte ou enfant), mais il est généralement compris entre trois et neuf.

Le tube 4 du dispositif selon l'invention peut être réalisé en toute matière déjà utilisée dans les sondes respiratoires, par exemple en un chlorure de polyvinyle, avec un éventuel revêtement de silicone ou en acier permettant les injections à pression élevée.

Bien entendu, les dimensions du dispositif selon l'invention peuvent être très variables, essentiellement en fonction de la voie de mise en place du tube et de la taille du patient, qui peut être un adulte, un enfant, un nourrisson ou un prématuré.

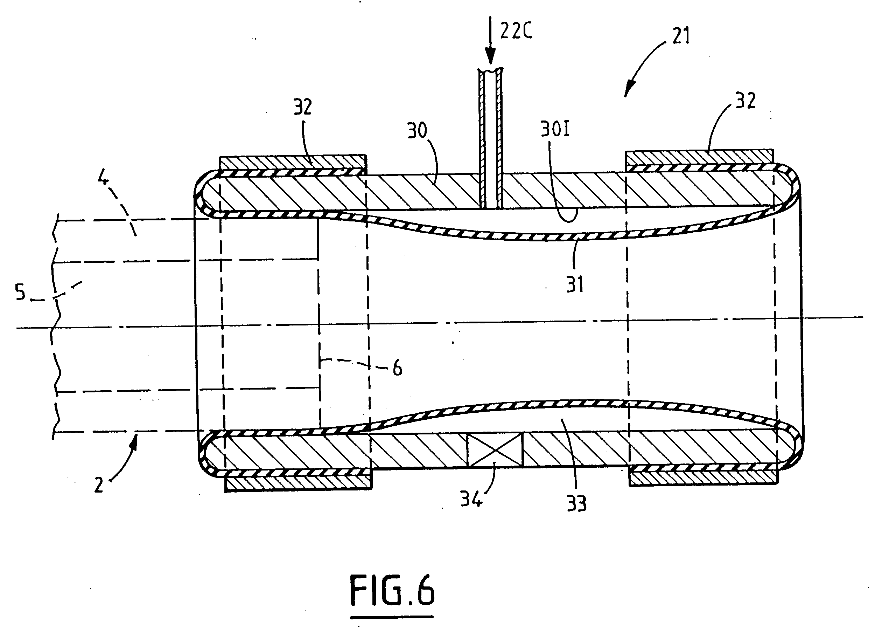

Sur la figure 6, on a représenté une variante de réalisation permettant d'utiliser un tube respiratoire 4 du commerce. Dans ce mode de réalisation, la vanne commandée 21 est solidaire d'un embout tubulaire rigide 30 pouvant être emboîté sur l'extrémité proximale 2 dudit tube. Dans l'exemple représenté sur la figure 6, la vessie 25 de la figure 4 a été remplacée par une portion de tube souple et élastique 31, disposée à l'intérieur de l'embout 30 et repliée à ses extrémités sur la paroi externe de celui-ci, où elle est maintenue par des bagues externes 32. Le conduit de gonflage 22C débouche dans la cavité 33 formée entre la paroi interne 30I de l'embout 30 et ladite portion 31 de tube souple et élastique. On comprendra aisément que la vanne de la figure 6 fonctionne de façon identique à celle de la figure 4. Une vanne d'échappement tarée 34 est prévue pour éventuellement mettre en communication la cavité 33 et l'atmosphère extérieure.

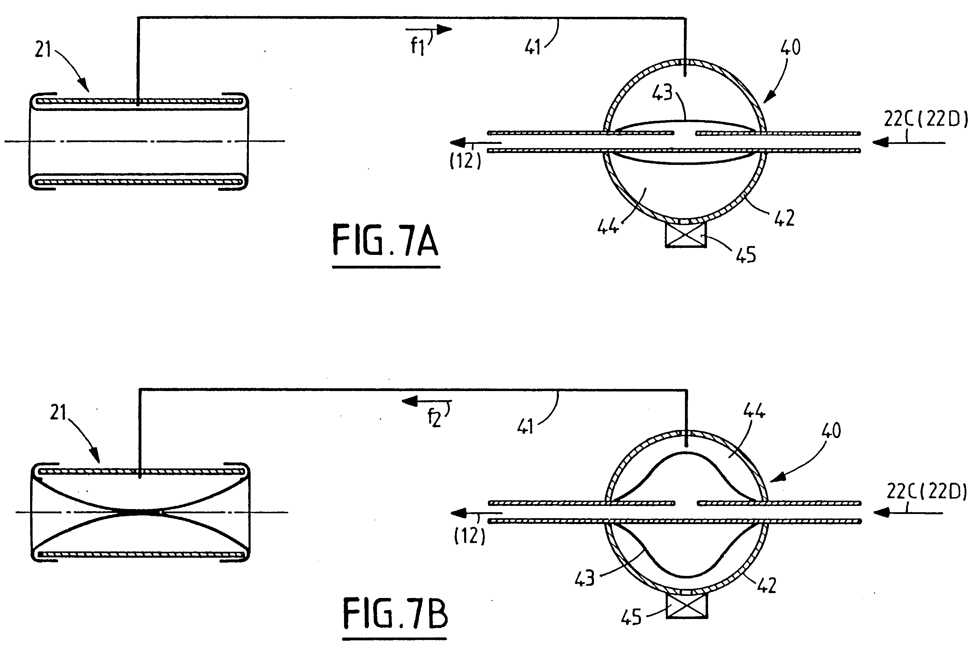

Le dispositif de gonflage de sécurité de la vanne commandée 21 (figure 4 ou figure 6), montré schématiquement par les figures 7A et 7B, comporte une capacité tampon 40 reliée à la vessie 25 ou à la cavité 33 par l'intermédiaire d'une communication 41.

La capacité tampon 40 est par exemple constituée d'une enveloppe extérieure rigide 42 et d'une enveloppe intérieure gonflable 43, délimitant entre elles un espace 44 à volume variable. C'est cet espace à volume variable 44 qui est relié à la vessie 25 ou à la cavité 33 par l'intermédiaire de la communication 41. De plus, l'enveloppe intérieure élastique gonflable 43 est reliée au dispositif 22. Comme représenté sur les figures 7A et 7B, la vanne commandée 21 peut être reliée audit dispositif 22 par la liaison 22C (comme décrit précédemment) ou par la liaison 22D (variante).

Une vanne d'échappement tarée 45 est prévue sur l'enveloppe extérieure 42 pour éviter toute surpression dans l'espace 44 à volume variable.

Ainsi, l'introduction et l'évacuation du gaz de gonflage de ladite vanne commandée 21 résulte du déplacement bidirectionnel dans la communication 41 du gaz contenu dans l'espace 44 à valeur variable. Par exemple, l'espace 44, lorsque l'enveloppe intérieure 43 est dégonflée, a un volume égal au volume interne de la vessie 25 ou de la cavité 33, lorsque la vanne 21 est totalement fermée.

Lorsqu'aucun gaz de gonflage n'est adressé à la capacité tampon 40 (figure 7A), l'enveloppe intérieure 43 est dégonflée et l'élasticité de la vessie 25 ou de la paroi 31 de la vanne 21 chasse le gaz contenu dans celle-ci (voir la flèche f1) dans l'espace à volume variable 44, à travers la communication 41. La vanne 21 est alors en position ouverte.

En revanche, lorsque du gaz de gonflage arrive dans l'enveloppe intérieure 43, celle-ci se gonfle et chasse le gaz de l'espace 44 vers la vessie 25 ou la cavité 33, à travers la communication 41 (voir la flèche f2).

## Revendications

1. Dispositif d'assistance respiratoire comportant un tube (4) qui forme un canal principal (5) et qui est destiné à être relié par son extrémité distale (3) à une voie respiratoire d'un patient pour que ledit canal principal (5) relie, à l'extérieur, le système respiratoire dudit patient, ledit dispositif comportant de plus au moins un canal auxiliaire (8) permettant d'insuffler un jet gazeux respirable dans ledit système respiratoire et débouchant dans ledit canal principal (5) au voisinage de l'extrémité distale (7) de ce dernier,
caractérisé en ce qu'il comporte une vanne commandée (21) susceptible d'obturer au moins partiellement l'extrémité proximale dudit canal principal (5), au moins pendant l'insufflation dudit jet gazeux.

2. Dispositif selon la revendication 1,
caractérisé en ce que ladite vanne commandée (21) forme un ensemble monolithique avec ledit tube (4).

3. Dispositif selon la revendication 1,
caractérisé en ce que ladite vanne commandée (21) est rapportée sur ledit tube (4).

4. Dispositif selon la revendication 3,
caractérisé en ce que ladite vanne commandée (21) est solidaire d'un embout (30) susceptible d'être emboîté sur l'extrémité proximale (2) dudit tube (4).

5. Dispositif selon l'une des revendications 1 à 4,
caractérisé en ce que ladite vanne commandée (21) comporte une enceinte étanche (25) à section toroïdale, disposée au voisinage de l'extrémité proximale dudit canal principal (5) et comportant au moins une paroi interne souple et élastique (25I) qui, en se dilatant ou en se rétractant en réponse à l'introduction ou à l'évacuation d'un gaz de gonflage dans ladite enceinte étanche, commande la section de passage dudit canal principal (5).

6. Dispositif selon l'une des revendications 1 à 5,
caractérisé en ce que le bord libre (18) de l'extrémité proximale dudit tube (4) comporte au moins une échancrure (27).

7. Dispositif selon l'une des revendications 1 à 6,
caractérisé en ce qu'il comporte au moins une prise de pression (20, 20A), disposée du côté de l'extrémité distale (3) dudit tube (4).

8. Dispositif selon la revendication 7,
caractérisé en ce que ladite prise de pression est formée par l'orifice (20A) par lequel un canal auxiliaire (20), ménagé dans ladite paroi dudit tube (4), débouche au voisinage de l'extrémité distale (19) dudit tube.

9. Dispositif selon l'une des revendications 7 ou 8,
caractérisé en ce qu'il comporte des moyens (22) recevant la pression prélevée par ladite prise de pression (20A) et susceptibles d'imposer l'ouverture à ladite vanne commandée (21) pour dégager ledit canal principal (5).

10. Dispositif selon l'une des revendications 1 à 9,
caractérisé en ce qu'il comporte une vanne d'échappement tarée (24), disposée à l'extrémité proximale dudit tube (4), du côté opposé au bord libre (18) de l'extrémité proximale (2) par rapport à ladite vanne commandée (21).

11. Dispositif selon l'une des revendications 5 à 10,
caractérisé en ce que l'introduction et l'évacuation du gaz de gonflage de ladite enceinte étanche résulte du déplacement bidirectionnel d'un volume de gaz approprié contenu dans une capacité tampon à volume variable commandable.

12. Dispositif selon l'une des revendications 1 à 10,
caractérisé en ce qu'au moins l'extrémité distale dudit canal auxiliaire (8) débouchant dans le canal principal (5) est parallèle à celui-ci et en ce que, en regard de l'orifice distal (17) dudit canal auxiliaire (8), sont prévus des moyens (14b) de déflexion dudit jet de gaz respirable de ventilation vers l'intérieur dudit canal principal (5).

## Claims

1. A device for respiratory assistance, comprising a tube (4) which forms a main channel (5) and which is intended to be connected via its distal end (3) to the respiratory tract of a patient so that said main channel (5) connects the respiratory system of said patient to the outside, with said device moreover comprising at least one auxiliary channel (8) permitting the insufflation of a respirable gaseous jet into said respiratory system and opening into said main channel (5) in the vicinity of the distal end (7) of the latter, characterized in that it comprises a controlled valve (21) capable of closing at least partially the proximal end of said main channel (5), at least during insufflation of said gaseous jet.

2. The device as claimed in claim 1, characterized in that said controlled valve (21) forms a single-piece assembly with said tube (4).

3. The device as claimed in claim 1, characterized in that said controlled valve (21) is attached to said tube (4).

4. The device as claimed in claim 3, characterized in that said controlled valve (21) is integral with a connection piece (30) capable of being engaged on the proximal end (2) of said tube (4).

5. The device as claimed in one of claims 1 to 4, characterized in that said controlled valve (21) comprises a sealed enclosure (25) of toroidal cross section arranged in the vicinity of the proximal end of said main channel (5) and comprising at least one flexible and elastic inner wall (25I) which, by expanding or retracting in response to the introduction or the removal of an inflating gas into or out of said sealed enclosure, controls the cross section of flow of said main channel (5).

6. The device as claimed in one of claims 1 to 5, characterized in that the free edge (18) of the proximal end of said tube (4) comprises at least one notch (27).

7. The device as claimed in one of claims 1 to 6, characterized in that it comprises at least one pressure tap (20, 20A) arranged toward the distal end (3) of said tube (4).

8. The device as claimed in claim 7, characterized in that said pressure tap is formed by the orifice (20A) via which an auxiliary channel (20), formed in said wall of said tube (4), opens out in the vicinity of the distal end (19) of said tube.

9. The device as claimed in either of claims 7 and 8, characterized in that it comprises means (22) receiving the pressure taken via said pressure tap (20A) and capable of forcing said controlled valve (21) to open in order to free said main channel (5).

10. The device as claimed in one of claims 1 to 9, characterized in that it comprises a calibrated escape valve (24) arranged at the proximal end of said tube (4), at the side opposite the free edge (18) of the proximal end (2) in relation to said controlled valve (21).

11. The device as claimed in one of claims 5 to 10, characterized in that the introduction and removal of the inflating gas of said sealed enclosure results from the bidirectional displacement of a suitable volume of gas, contained in a buffer area of controllable variable volume.

12. The device as claimed in one of claims 1 to 10, characterized in that at least the distal end of said auxiliary channel (8) opening into the main channel (5) is parallel thereto, and in that there are provided, facing the distal orifice (17) of said auxiliary channel (8), means (14b) for deflecting said jet of respirable ventilation gas toward the inside of said main channel (5).

## Patentansprüche

1. Vorrichtung zur Unterstützung der Atmung umfassend ein Rohr (4), das einen Hauptkanal (5) bildet und das dafür vorgesehen ist, mit seinem distalen Ende (3) mit einem Atemweg eines Patienten verbunden zu sein, damit der Hauptkanal (5) das Atmungssystem des Patienten mit der Außenwelt verbindet, wobei die Vorrichtung weiterhin wenigstens einen Hilfskanal (8) umfaßt, der es erlaubt, einen Strahl atembaren Gases in das Atmungssystem einzublasen und der in den Hauptkanal (5) in der Nähe des distalen Endes (7) mündet, dadurch gekennzeichnet, daß sie ein geregeltes Ventil (21), geeignet für das wenigstens teilweise Verschließen des proximalen Endes des Hauptkanals (5), wenigstens während der Einblasung des Gasstrahls umfaßt.

2. Vorrichtung nach Anspruch 1, dadurch gekennzeichnet, daß das gesteuerte Ventil (21) eine monolithische Einheit mit der Röhre (4) bildet.

3. Vorrichtung nach Anspruch 1, dadurch gekennzeichnet, daß das geregelte Ventil (21) auf die Röhre (4) aufgesteckt ist.

4. Vorrichtung nach Anspruch 3, dadurch gekennzeichnet, daß das geregelte Ventil (21) aus einem einstückigen Ansatz (30) gebildet ist, der dafür geeignet ist, an das proximale Ende (2) der Röhre (4) angefügt zu werden.

5. Vorrichtung nach einem der Ansprüche 1 bis 4, dadurch gekennzeichnet, daß das geregelte Ventil (21) eine luftdichte Einfassung (25) mit torusförmigem Querschnitt enthält, wobei es in der Nähe des proximalen Endes des Hauptkanals (5) angeordnet ist und wobei es wenigstens eine nachgiebige und elastische Innenwand (25I) umfaßt, die beim Ausweiten oder beim Zusammenziehen in Reaktion auf die Einführung oder Evakuation eines Treibgases in die dichte Einfassung, den Querschnitt des Hauptkanals (5) regelt.

6. Vorrichtung nach einem der Ansprüche 1 bis 5, dadurch gekennzeichnet, daß der freie Rand (18) der proximalen Öffnung der Röhre (4) wenigstens eine Aussparung (27) aufweist.

7. Vorrichtung nach einem der Ansprüche 1 bis 6, dadurch gekennzeichnet, daß sie wenigstens eine Druckanschlußstelle (20, 20A) aufweist, die gegenüber dem distalen Ende (3) der Röhre (4) angeordnet ist.

8. Vorrichtung nach Anspruch 7, dadurch gekennzeichnet, daß die Druckanschlußstelle durch die Öffnung (20A) ausgebildet ist, durch welche ein Hilfskanal (20), der in der Wandung der Röhre (4) ausgespart ist, in der Nähe des distalen Endes (19) der Röhre mündet.

9. Vorrichtung nach einem der Ansprüche 7 oder 8, dadurch gekennzeichnet, daß sie Mittel (22) aufweist, die den vorherrschenden Druck durch die Druckanschlußstelle (20A) aufnehmen und geeignet sind, dem geregelten Ventil (21) die Öffnung zu erzwingen, um den Hauptkanal (5) frei zu machen.

10. Vorrichtung nach einem der Ansprüche 1 bis 9, dadurch gekennzeichnet, daß sie ein geeichtes Auslaßventil (24) aufweist, das am proximalen Ende der Röhre (4) in Bezug auf das geregelte Ventil (21) gegenüber dem freien Rand (18) des proximalen Endes (2) angeordnet ist.

11. Vorrichtung nach einem der Ansprüche 5 bis 10, dadurch gekennzeichnet, daß die Einführung und Evakuation des Druckgases bei der luftdichten Einfassung durch den bidirektionalen Strom eines geeigneten Gasvolumens erfolgt, das in einer Pufferkapazität mit variablem, veränderbarem Volumen aufgenommen ist.

12. Vorrichtung nach einem der Ansprüche 1 bis 10, dadurch gekennzeichnet, daß wenigstens das distale Ende des Hilfskanals (8), das in den Hauptkanal (5) mündet, parallel zu ihm verläuft und dadurch, daß im Hinblick auf die distale Öffnung (17) des Hilfskanals (8) Mittel zur Ablenkung (14b) des Strahls atembaren Gases für die Atmung gegen das Innere des Hauptkanals (5) vorgesehen sind.
